# EUROPÄISCHE PATENTANMELDUNG

(11) **EP 0 902 012 A2**
(43) Veröffentlichungstag der Anmeldung: **17.03.1999**
(21) Anmeldenummer: 98116076.5
(22) Anmeldetag: 26.08.1998
(51) Int. Cl.: C07C 253/00, C07C 255/07

(54) **Verfahren zur Herstellung aliphatischer, ungesättigter Nitrile**

(30) Priorität: 04.09.1997 DE 19738576
(71) Anmelder: BASF AKTIENGESELLSCHAFT, 67056 Ludwigshafen (DE)
(72) Erfinder: Kramer, Andreas, Dr., 67251 Freinsheim (DE); Siegel, Wolfgang, Dr., 67117 Limburgerhof (DE); Henningsen, Michael, Dr., 67227 Frankenthal (DE); Grosch, Georg Heinrich, Dr., 67098 Bad Dürkheim (DE)

(57) **Zusammenfassung**

Verfahren zur Herstellung von ungesättigten Nitrilen der Formel I 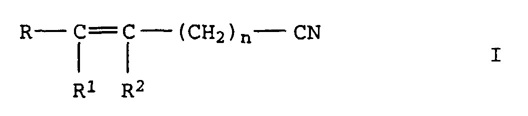 in der R, R¹ und R² unabhängig voneinander Alkyl- oder Alkenylreste mit 1 bis 20 C-Atomen oder Cycloalkyl- oder Cycloalkenylreste mit 3 bis 12 C-Atomen bedeuten, wobei jeweils 2 der Reste R, R¹ und R² Bestandteile eines gesättigten oder ungesättigten Rings sein können und wobei R¹ und R² auch für ein Wasserstoffatom stehen können und n die Werte 0 oder 1 bis 20 bedeutet, durch Dehydratisierung von Oximen der Formel II 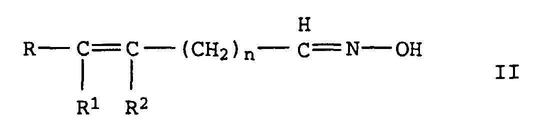 in der R, R¹, R² und n die oben angegebenen Bedeutungen haben,
dadurch gekennzeichnet, daß man die Dehydratisierung in der Gas- oder Flüssigphase bei Temperaturen von 150 bis 300°C über einem heterogenen Katalysator durchführt, der aus einem oxidischen, carbidischen oder nitridischem Träger besteht, der mit basischen Oxiden und/oder Hydroxiden oder mit Verbindungen, die unter den Reaktionsbedingungen in diese übergehen, dotiert ist, oder der aus den basischen Oxiden besteht.

## Beschreibung

Die Erfindung betrifft die Herstellung aliphatischer, ungesättigter Nitrile aus ungesättigten Aldoximen durch katalytische Dehydratisierung in der Gas- oder Flüssigphase über oxidischen, carbidischen oder nitridischen Trägerkatalysatoren, die mit Metalloxid- oder -hydroxidbasen dotiert sind, oder über basischen Metalloxid-Vollkontakten.

Die Dehydratisierung von Aldoximen zu Nitrilen ist in der Fachliteratur eingehend beschrieben. Eine zusammenfassende Darstellung mehrerer Methoden unter Verwendung verschiedener Reagenzien findet man bei A.J. Fatiadi in The Chemistry of Triple Bonded Functional Groups, Supplement D, Part 2, 1057-1303, S. Patai und Z. Rappoport, Eds. John-Wiley New York, 1983.

Von besonderem Interesse ist eine neuere Methode gemäß WO 9302046-A1, bei der Citraloxim (3,7-Dimethyl-octa-2,6-dienaloxim) mit Kaliumhydroxid in Toluol unter Wasserauskreisen dehydratisiert wird. Diese Methode hat allerdings den Nachteil, daß sie weniger gut in großtechnischem Maßstab durchzuführen ist, wofür Verfahren über heterogenen Katalysatoren bevorzugt werden.

Die bisher beschriebenen Dehydratisierungen über heterogenen Katalysatoren, das ist in der Regel in der Gasphase, sind jedoch noch nicht befriedigend.

So sind bei der von M.N. Rao et al. in Org. Prep. Proced. Int. 21, 230-232 (1989) beschriebenen Gasphasen-Dehydratisierung aromatischer und aliphatischer Aldoxime über Cs-X-Zeolithen hohe Temperaturen von über 300°C erforderlich.

Weiterhin ist in Houben-Weyl, Bd. 8 S. 326 ff, über die Dehydratisierung von Oximen zu Nitrilen an Aluminium- oder Thoriumoxiden bei hohen Temperaturen, das ist über 340°C, referiert.

Zur Vermeidung dieser hohen Temperaturen wurde darauf von H.M. Meshram in Synthesis (1992), 943-944 vorgeschlagen, für die Gasphasendehydratisierung von aromatischen oder gesättigten aliphatischen Aldoximen Montmorillonit KSF (das ist ein saure Zentren aufweisender Ton) als Katalysator zu verwenden. Damit verläuft die Umsetzung zwar bei niedrigeren Temperaturen, jedoch macht die unzureichende Raum-Zeit-Ausbeute dieses Verfahren wirtschaftlich unattraktiv.

Es bestand daher die Aufgabe, ein Verfahren vorzuschlagen, das die Dehydratisierung von ungesättigten Aldoximen über heterogenen Katalysatoren bei relativ niedrigen Temperaturen, kurzer Verweilzeit und in guten Ausbeuten erlaubt.

Diese Aufgabe wurde erfindungsgemäß gelöst mit einem Verfahren zur Herstellung von ungesättigten Nitrilen der Formel I 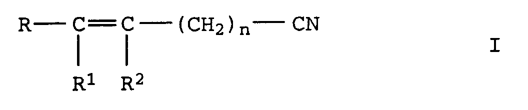 in der R, R¹ und R² unabhängig voneinander Alkyl- oder Alkenylreste mit 1 bis 20 C-Atomen oder Cycloalkyl- oder Cycloalkenylreste mit 3 bis 12 C-Atomen bedeuten, wobei jeweils 2 der Reste R, R¹ und R² Bestandteile eines gesättigten oder ungesättigten Rings sein können und wobei R¹ und R² auch für ein Wasserstoffatom stehen können und n die Werte 0 oder 1 bis 20, insbesondere 0 oder 1 bis 7, bedeutet, durch Dehydratisierung von Oximen der Formel II 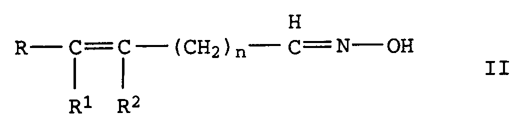 in der R, R¹, R² und n die oben angegebenen Bedeutungen haben,
dadurch gekennzeichnet, daß man die Dehydratisierung in der Gas- oder Flüssigphase bei Temperaturen von 150 bis 300°C über einem heterogenen Katalysator durchführt, der aus einem oxidischen, carbidischen oder nitridischem Träger besteht, der mit basischen Oxiden und/oder Hydroxiden oder mit Verbindungen, die unter den Reaktionsbedingungen in diese übergehen, dotiert ist, oder der aus den basischen Oxiden besteht.

Als Ausgangsmaterialien der Formel II kommen vor allem Verbindungen mit n = 0 bzw. n = 1 bis n = 7 in Betracht. Dies sind z.B. Angelicaoxim, Aleprinoxim, α,β-Apocitronellaloxim, Bergamotenoxim, Brenzterebinoxim, Campholenoxim, Citronellaloxim, Citraloxim, Chrysanthemoxim, Cyclocitraloxim, Cyclolavandulaloxim, Faranaloxim, Farnesaloxim, Isolauranaloxim, Ikemaoxim, Myrthenaloxim, Phellandrinoxim, Safranaloxim und Sorbinaloxim.

Vorzugsweise verwendet man solche Verbindungen der Formel I, in der n = 0 bedeutet.

Im einzelnen sind Angelicaoxim, Bergamotenoxim, Cyclolavandulaloxim, Citraloxim, Farnesaloxim, Ikemaoxim, Isolauranaloxim, Phellandrinoxim und Sorbinaloxim zu nennen. Insbesondere kommt Citraloxim in Betracht, aus dem Geranonitril entsteht.

Als Katalysatoren kommen allgemein mit basischen Metalloxiden oder -hydroxiden dotierte Trägerkatalysatoren in Betracht. Unter Dotierung wird dabei nicht nur das Aufbringen der Metalloxide oder -hydroxide verstanden, sondern auch der Ionenaustausch gegen z.B. Alkali- oder Erdalkaliionen bei für den Ionenaustausch geeigneten Trägermaterialien.
Die Träger sind, wie meist üblich, oxidischer, carbidischer oder nitridischer Natur. Bevorzugt werden jedoch als Träger Metalloxide von Metallen der Gruppen Ib bis VIIb, VIII und IIIa bis Va, insbesondere IIIb bis VIIb, VIII, IIIa und IVa sowie deren Mischungen.
Dabei ist es unkritisch, ob die Metalloxide röntgenographisch kristallin oder amorph sind.

Im einzelnen sind vor allem Aluminiumoxid, Siliciumdioxid, Titandioxid und Zirkondioxid in beliebigen Modifikationen zu nennen. Die Träger haben im allgemeinen eine BET-Oberfläche von 0,1 bis 500, vorzugsweise 1 bis 400 m²/g.

Zur Entfaltung der Dehydratisierungsaktivität im Bereich unterhalb 300°C müssen die Katalysatorträger mit basischen Metalloxiden oder -hydroxiden, oder mit Metallverbindungen, die diese unter Reaktionsbedingungen bilden, dotiert sein.

Diese Metallionen zur Dotierung der oxidischen Massen werden ausgewählt aus der Gruppe Ia, IIa bis VIII und IIb bis Vb, insbesondere IIb oder IIIb, sowie aus der Gruppe der Lanthaniden bestehend aus La, Ce, Pr, Nd, Sm, Eu, Gd, Tb, Dy, Ho, Er, Tm, Yb, Lu sowie Mischungen daraus. Bevorzugt sind die Metallionen der Gruppe Ia bestehend aus Li, Na, K, Rb, Cs und der Gruppe IIa bestehend aus Be, Mg, Ca, Sr, Ba. Der Gehalt an basischen Oxiden/Hydroxiden, bezogen auf den Katalysator und berechnet als Oxide, liegt zwischen 0,1 und 50 Gew.-%, bevorzugt 0,2 und 40 Gew.-%, besonders bevorzugt 0,3 und 30 Gew.-% und insbesondere zwischen 1 bis 10 Gew.-%.

Die Dotierung der oxidischen Grundmassen kann durch an sich bekannte Verfahren, wie Imprägnieren, Tränken oder Ionenaustausch, erfolgen. Die Katalysatoren werden nach der Dotierungsprozedur in der Regel getrocknet und anschließend kalziniert. Die Kalziniertemperatur liegt in der Regel zwischen 150 und 800°C, bevorzugt zwischen 150 und 700°C, und besonders bevorzugt zwischen 300 und 650°C.

Die Dehydratisierung der ungesättigten Aldoxime zu den entsprechenden Nitrilen wird im allgemeinen bei 0,5 bis 5 bar und Temperaturen von 150 bis 300°C, bevorzugt 200 bis 250°C, ansatzweise oder in kontinuierlicher Form durchgeführt, wobei die kontinuierliche Arbeitsweise besonders bevorzugt wird. Für die kontinuierliche Umsetzung eignet sich die Umsetzung der Aldoxime am Festbett in der Gasphase oder in der Flüssigphase sowie in der Gasphase am Wirbelbett.

Die erfindungsgemäße Umsetzung kann in Gegenwart oder Abwesenheit von Lösungsmitteln durchgeführt werden.
Für die Dehydratisierung über einem fest angeordneten Katalysator in der Gasphase verwendet man vor allem unpolare Lösungsmittel, die unter den Reaktionsbedingungen dampfförmig sind. Geeignete Lösungsmittel sind z.B. Kohlenwasserstoffe mit bis zu 12 C-Atomen, wie Hexan, Heptan oder Octan.
Für die Umsetzung in der Flüssigphase kommen Losungsmittel in Betracht, die unter den Reaktionstemperaturen flüssig sind. Dies sind vor allem höher siedende Kohlenwasserstoffe wie Paraffinöle und Weißöle mit einem Siedepunkt z.B. von über 300°C.

Auch polare mittel- und hochsiedende Lösungsmittel, die sich durch Destillation vom Produkt abtrennen lassen, kommen in Betracht, wie N-Methylpyrrolidon, N-Methylmorpholin, Dimethylpropylenharnstoff oder Dimethylethylenharnstoff.

Die erfindungsgemäß herzustellenden ungesättigten Nitrile der Formel II werden in ausgezeichneter Raumzeitausbeute bei kurzen Verweilzeiten im Reaktor und sehr guten Selektivitäten bei gutem Umsatz erhalten.

### Beispiele

### Allgemeine Durchführung

Die Dehydratisierung von Citraloxim wurde in einer Gasphasenapparatur im Labormaßstab unter folgenden Standardbedingungen durchgeführt:
Aus einem 100-ml-Tropftrichter wurden zwischen 13 und 26 ml/h Zulauf in Form von reinem Citraloxim (E,Z-Gemisch) oder als Lösung (1:1) in N-Methylpyrrolidon oder n-Hexan mit einer Dosierpumpe in die Vorheizzone des Gasphasenreaktors (Gesamtlänge 500 mm, davon 200 mm Vorheizstrecke, 300 ml Reaktionszone, Durchmesser 30 mm) gefördert. Die Beheizung des Reaktors erfolgte mit einem geteilten elektrisch beheizten Quarzofen, wobei die Vorheizstrecke mit 500 W auf ca. 210°C und die Reaktionszone mit 1000 W auf 250°C erhitzt wurde. Die Temperaturregelung und -kontrolle wurde mit Pt-100-Thermoelementen in einer Thermohülse (Durchmesser 6,5 mm) in der Längsachse des Reaktors durchgeführt. Unter regelbarem Stickstoffstrom (5 bis 20 l/h, gemessen anhand eines Rotameters) wurde das Reaktionsgut an 100 ml Katalysator (Stränge) umgesetzt. Der gasförmige Reaktionsaustrag wurde an einem Energiekugelkühler mit nachgeschaltetem CO₂-Kühler kondensiert und in einem Vorlagegefäß aufgefangen. Die Aufarbeitung der Reaktionsausträge zur Isolierung des Geranonitrils erfolgt destillativ.

Unter Variation des Katalysators wurde die in Tabelle 1 und Variation der Temperatur die in Tabelle 2 angegebenen Ergebnisse erhalten.

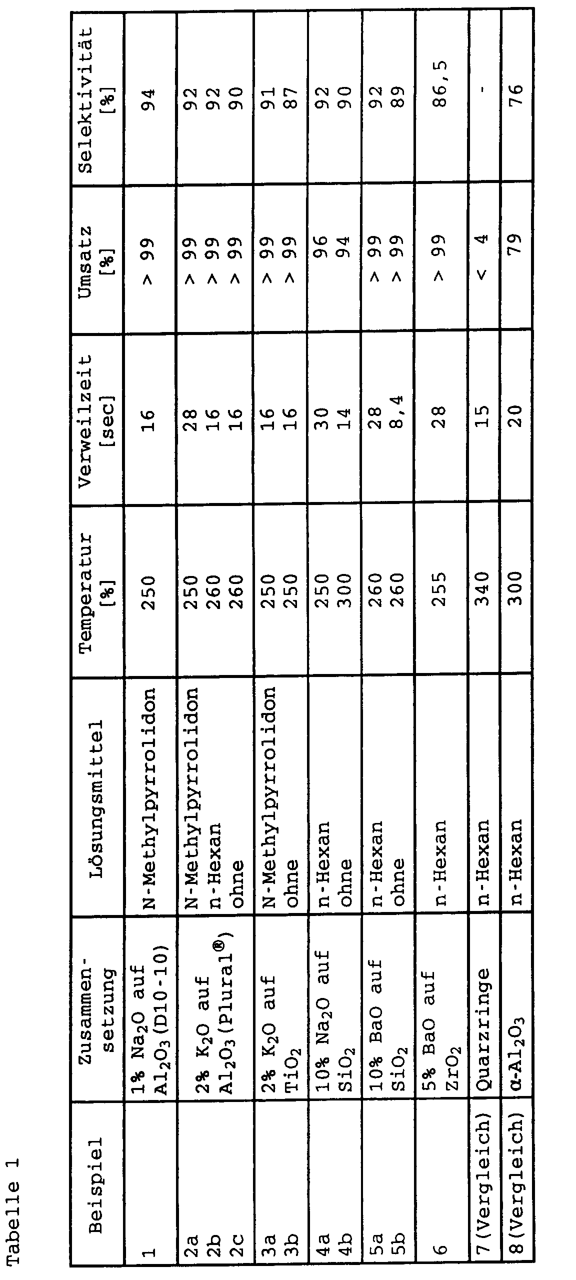

**Tabelle 2**

| Beispiel | Temperatur [°C] | Verweilzeit [sec] | Selektivität [%] |
|---|---|---|---|
| 1a | 250 | 16 | 92 |
| 1b | 300 | 15 | 90 |
| 1c | 350 | 14 | 72 |
| 1d | 400 | 13 | 32 |

### Beispiel 9 (Dehydratisierung in der Flüssigphase)

In einer Apparatur wie sie in Beispiel 1 beschrieben ist, wurde eine Mischung aus 10 Gew.-% Citraloxim in Paraffinöl an einem Katalysator (5 % BaO auf ZrO₂) unter Normaldruck umgesetzt. Die Temperatur des Vorheizers betrug 150 - 220°C die Reaktortemperatur 220°C. Der Zulauf betrug 239 ml/h, so daß bei einer Katalysatorbelastung von 0,95 kg/l·h ein Umsatz 98 % erreicht wurde. Aus dem aus dem Reaktor austretenden Reaktionsgut wurde auf einem Wischblattverdampfer das Geranonitril vom Paraffinöl abgetrennt. Die Ausbeute des so erhaltenen Nitrils, das destillativ weiter gereinigt werden kann, war größer 90 %.

## Patentansprüche

1. Verfahren zur Herstellung von ungesättigten Nitrilen der Formel I 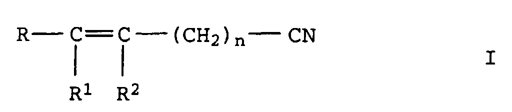 in der R, R¹ und R² unabhängig voneinander Alkyl- oder Alkenylreste mit 1 bis 20 C-Atomen oder Cycloalkyl- oder Cycloalkenylreste mit 3 bis 12 C-Atomen bedeuten, wobei jeweils 2 der Reste R, R¹ und R² Bestandteile eines gesättigten oder ungesättigten Rings sein können und wobei R¹ und R² auch für ein Wasserstoffatom stehen können und n die Werte 0 oder 1 bis 20 bedeutet, durch Dehydratisierung von Oximen der Formel II 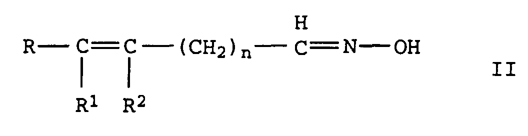 in der R, R¹, R² und n die oben angegebenen Bedeutungen haben,
dadurch gekennzeichnet, daß man die Dehydratisierung in der Gas- oder Flüssigphase bei Temperaturen von 150 bis 300°C über einem heterogenen Katalysator durchführt, der aus einem oxidischen, carbidischen oder nitridischen Träger besteht, der mit basischen Oxiden und/oder Hydroxiden oder mit Verbindungen, die unter den Reaktionsbedingungen in diese übergehen, dotiert ist, oder der aus den basischen Oxiden besteht.

2. Verfahren gemäß Anspruch 1, dadurch gekennzeichnet, daß man Katalysatoren verwendet, die als Träger Metalloxide der Elemente der Gruppen Ib bis VIIb und VIII oder IIIa bis Va des Periodensystems der Elemente oder Gemische dieser Metalloxide enthalten, die mit 0,1 bis 50 Gew.-%, bezogen auf den Katalysator und berechnet als Oxide, an basischen Oxiden oder Hydroxiden oder Verbindungen, die unter den Reaktionsbedingungen basische Oxide oder Hydroxide bilden, von Elementen der Gruppen Ia, IIa, IIb bis Vb und/oder Lanthaniden dotiert sind.

3. Verfahren gemäß Anspruch 1, dadurch gekennzeichnet, daß man Katalysatoren verwendet, die als Träger Metalloxide der Elemente der Gruppen IIb bis VIIb oder VIII oder IIIa bis IVa des Periodensystems der Elemente oder Gemische dieser Metalloxide enthalten und mit 0,2 bis 40 Gew.-%, bezogen auf den Katalysator und berechnet als Oxide, an Alkali- oder Erdalkalioxiden oder -hydroxiden oder Verbindungen, die unter den Reaktionsbedingungen in diese übergehen, dotiert sind.

4. Verfahren gemäß Anspruch 1, dadurch gekennzeichnet, daß man Katalysatoren verwendet, die 0,3 bis 30 Gew.-%, bezogen auf den Katalysator und berechnet als Oxide, Alkali- oder Erdalkalioxide oder -hydroxide auf einem oxidischen Träger enthalten, ausgewählt aus der Gruppe bestehend aus Aluminiumoxid, Siliciumdioxid, Titandioxid und Zirkondioxid.

5. Verfahren gemäß Anspruch 1, dadurch gekennzeichnet, daß man Katalysatoren verwendet, die 1 bis 10 Gew.-% (berechnet als Oxide) an Alkali- oder Erdalkalioxiden oder -hydroxiden enthalten.

6. Verfahren gemäß Anspruch 1, dadurch gekennzeichnet, daß man als oxidische Katalysatoren geformte Katalysatoren verwendet, ausgewählt aus der Gruppe bestehend aus Aluminiumoxid, Siliciumdioxid, Titandioxid und Zirkondioxid, die mit einer Lösung einer alkalischen Alkali- oder Erdalkaliverbindung getränkt und bei Temperaturen von 300 bis 650°C calciniert worden sind.

7. Verfahren gemäß Anspruch 1, dadurch gekennzeichnet, daß man die Dehydratisierung in Gegenwart eines unter den Reaktionsbedingungen dampfförmig vorliegenden Lösungsmittels in der Gasphase durchführt.

8. Verfahren gemäß Anspruch 1, dadurch gekennzeichnet, daß man die Dehydratisierung in Gegenwart eines unter den Reaktionsbedingungen flüssigen Lösungsmittels in der Flüssigphase durchführt.

9. Verfahren gemäß Anspruch 1, dadurch gekennzeichnet, daß man die Reaktion bei Temperaturen von 200 bis 250°C und Drücken von 0,5 bis 5 bar durchführt.

10. Verfahren gemäß Anspruch 1, dadurch gekennzeichnet, daß man Geranonitril durch Dehydratisierung von Citraloxim herstellt.
